# EUROPEAN PATENT SPECIFICATION

(11) **EP 1 348 696 B1**
(45) Date of publication and mention of the grant of the patent: **01.02.2012**
(21) Application number: 02006881.3
(22) Date of filing: 26.03.2002
(51) Int. Cl.: C07D 207/26, A61K 31/4015

(54) **Use of optically active clausenamides for the preparation of pharmaceutical compositions, pharmaceutical compositions containing the same**
Verwendung von optisch aktiven Clausenamiden zur Herstellung einer pharmazeutischen Zusammensetzung und deren pharmazeutische Zusammensetzungen
Utilisation de clausenamides optiquement actifs pour la préparation de compositions pharmaceutiques, compositions pharmaceutiques les contenant

(43) Date of publication of application: 01.10.2003
(73) Proprietor: INSTITUTE OF MATERIA MEDICA, CHINESE ACADEMY OF MEDICAL SCIENCES, Beijing 10050 (CN)
(72) Inventor: Zhang, Juntian, Institute of Materia Medica,, No. 1 Xian Nong Tan St., Beijing 100050 (CN); Huang, Liang, Institute of Materia Medica,, No. 1 Xian Nong Tan St., Beijing 100050 (CN); Wu, Kemci, Institute of Materia Medica,, No. 1 Xian Nong Tan St., Beijing 100050 (CN); Chen, Shiming, Institute of Materia Medica,, No. 1 Xian Nong Tan St., Beijing 100050 (CN)
(74) Representative: TBK

(56) References cited:
- US-A- 4 879 390
- US-A- 5 132 433
- CHEMICAL ABSTRACTS, vol. 132, no. 11, 13 March 2000 (2000-03-13) Columbus, Ohio, US; abstract no. 131715, YAO, QING-QIANG ET AL: "Stereoselectivity of chiral clausenamide biotransformation" XP002203767 & ZHONGGUO YAOLIXUE YU DULIXUE ZAZHI (1999), 13(3), 214-216 ,
- CHEMICAL ABSTRACTS, vol. 129, no. 20, 16 November 1998 (1998-11-16) Columbus, Ohio, US; abstract no. 254812, LIU, SHAOLIN ET AL: "Difference between the effects of (-)clausenamide and (+)clausenamide on the synaptic transmission in the dentate gyrus of anesthetized rats" XP002203768 & YAOXUE XUEBAO (1998), 33(4), 254-258 ,
- CHEMICAL ABSTRACTS, vol. 112, no. 21, 21 May 1990 (1990-05-21) Columbus, Ohio, US; abstract no. 198860, CHENG, JIACHONG ET AL: "Separation of diastereoisomers-nuclausenamide and isoneoclausenamide and x-ray diffraction analysis" XP002203769 & CHIN. SCI. BULL. (1989), 34(11), 957-9 ,
- WANG J-Q ET AL: "ASYMMETRIC TOTAL SYNTHESIS OF (+)-(3R,4S,5R,7S)- NEOCLAUSENAMIDE" JOURNAL OF THE CHEMICAL SOCIETY, CHEMICAL SOCIETY. LETCHWORTH, GB, no. 2, 1996, pages 209-212, XP000916286 ISSN: 0368-1769

## Description

### Field of the invention

The present invention relates to some new optically active clausenamide derivatives, process of the preparation thereof, a pharmaceutical composition containing the same and their medical use, particularly their use in the respects of hypoxia protective, nootropic and neurodegenerative disease such as cerebral ischemia, Alzheimer disease and vascular dementia.

### Background of the invention

The average life span in Chinese population is now over 70 years of age.It increased by 100% as compared with before foundation of P.R. China.A scientific of study in abroad estimates that the proportion of the population over 65 years of age will increase to 18.8% by the 2025,when it will just exceed the proportion of children (18.6%). It means that one of 5population in about 20 years later will be senile. Alzheimer's disease(AD) and vascular damentia(VD) refer to the presenile and senile form of the disease. i.e. under age 65 vs age 65 and over. Therefore, with the aging of population, the incidence of AD and VD will certainly be increased. The old men and their related neurodegenerative diseases especially various dementia have to undergoing two kinds of death, firstly the psychological death and then physical death. This is a heavy load for not only patients but also families and society. The aging of population is considered as a disadvantage factor, next to the war, pestilence, farming and shortage of resource and energy, to society's development and stability.

There are many drugs for prevention and treatment of senile dementia, such as cerebral vasodilators which can increase cognition function through improvement of blood flow and energy. However, a real valuable cerebral vasodilator should have high selectivity with no effect on brain metabolism and no "blood steal" phenomenon , it needs also to have antiplatelet aggregation and antithrombosis actions. Calcium channel blocker nimodipine accords with some demands as mentioned above ,it acts on L-type but not the N-and T-type voltage dependent calcium channel. The drugs for central cholinergic system, the precoursor of acetyecholine(Ach) has weak therapeutic effect. Ach receptor agonists and cholinesterase inhibitors have certain sympmatically therapeutic effect, but its effect is short, and more important is that they can not affect athogenesis of dementia. Many neuropeptides and neurotrophine factors were considered as promising antidementia agents, but its clinical therapeutic effect is not good. The main reason for this is that these large molecules could not pass through the blood-brain barrier. Piracetam or 2-oxo -1 pyrolidine acetamide is usually considered as the prototype nootropic drug. In recent years, clinical studies indicated that the effect of piratcetam in improving memory impairment was mild or obscure. In addition, its mechanism of action is not fully understood, although it has been studied for nearly 30years.

Racemic clausenamide was isolated from the aqueous extract of leaves of Rutoceae Clausena lausium(Lour) Skeels and had been claimed to have pronounced cerebal hypoxia protective and antiamnestic effect. Its potential medical use and processes of preparation from the plant had been granted a patent right for Chinese Academy of Medical Sciences and Bayer AG(EU patent No. 072514,US patent No. 4879391 and Chinese patent No. 90107309). Neurobiochemical studies with (+) and (-) clausenamide were carried out in Liu, Shadin et al, YAOXUE XUEBAO (1998), 33(4), 254-58 Methods for chemical synthesis of racemic clausenamide also had been applied for patent protection (German patent No. 3927370, EP No. 0414120).

### Summary of the Invention

The object of the present invention is to provide new compounds for the prevention and/or treatment of neurodegenerative disease such as cerebral ischemia, Alzheimer disease and vascular dementia.

The present invention is directed to the use of an optically active chiral compound of Formula II, 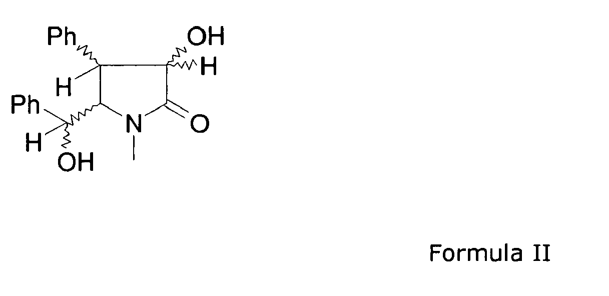 for the manufacture of a pharmacutical composition for the treatment of neurodegenerative diseases, wherein said derivative is selected from the group consisting of
3S4R5R6R (-) epiclauseanamide,
3R4S5S6S (+) epiclauseanamide,
3S4R5S6S (-) epineoclauseanamide
3R4S5R6R (+) epineoclauseanamide
3S4S5S6R (+) cisclauseanamide,
3R4R5R6S (-) cisclauseanamide,
3S4S5S6S (+) cisepiclauseanamide,
3R4R5R6R (-) cisepiclauseanamide,
3S4S5R6S (-) cisneoclauseanamide,
3R4R5S6R (+) cisneoclauseanamide,
3S4S5R6R (-) cisepineoclauseanamide and
3R4R5S6S (+) cisepineoclauseanamide.

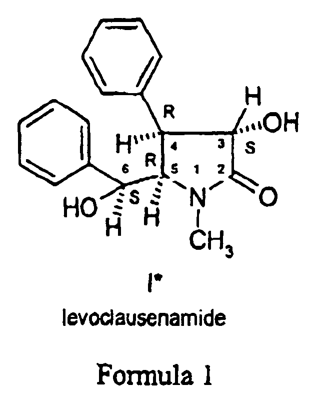

The invention provides to a pharmaceutical composition comprising some optically active compounds of formula II and pharmaceutically carrier or excipient.

The invention provides to a pharmaceutical composition for the prevention and/or treatment of neurodegenerative diseases comprising any one of the optically active compounds of formula II and pharmaceutically carrier or excipient.

The invention provides to a use of any one of the optically active compounds of formula II for the manufacture of medicament for the prevention and/or treatment of neurodeganerative diseases.

The invention provides a method for the prevention and/or treatment of neurodegenerative disease comprising administrating to a patient suffered with neurodegenerative disease or high risk of neurodegenrative disease an effective prevention and/or treatment amount of any one of the optically active compounds of formula II.

It is described a process for the preparation of levoclausenamide, which characterized in that:
1. De novo synthesis by using intramoleuclar inductive asymmetrical Dargen reaction to prepare compound (2*); 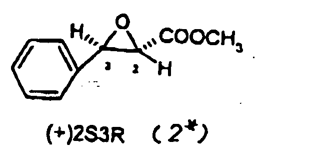 ;or
2. Biocatalystic resolution of said compound (2*); or
3. Chemical resolution of the racemic clausenamidone of formula (5*)

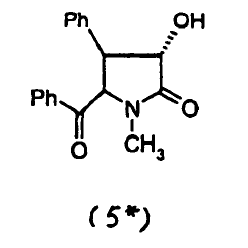

### Detailed Description of the Invention

The present invention is directed to the use of an optically active chiral compound of Formula II, 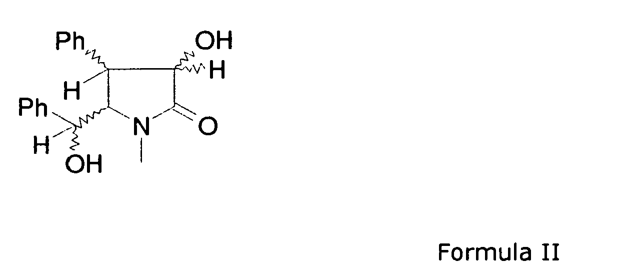 for the manufacture of a pharmacutical composition for the treatment of neurodegenerative diseases, wherein said derivative is selected from the group consisting of
3S4R5R6R (-) epiclauseanamide,
3R4S5S6S (+) epiclauseanamide,
3S4R5S6S (-) epineoclauseanamide
3R4S5R6R (+) epineoclauseanamide
3S4S5S6R (+) cisclauseanamide,
3R4R5R6S (-) cisclauseanamide,
3S4S5S6S (+) cisepiclauseanamide,
3R4R5R6R (-) cisepiclauseanamide,
3S4S5R6S (-) cisneoclauseanamide,
3R4R5S6R (+) cisneoclauseanamide,
3S4S5R6R (-) cisepineoclauseanamide and
3R4R5S6S (+) cisepineoclauseanamide.

The present invention also relates to a pharmaceutical composition comprising an optically active compound of formula II as an active ingredient and suitable pharmaceutically acceptable carrier or excipient. The an optically active compound of formula II are administrated alone or in the form of a pharmaceutical composition containing the same. Administration route may be intestinal or parenteral, such as oral, intramuscular, substcutaneous, transdermally, intransally, intraperitoneally, topically etc, said pharmaceutical preparations may formulated by mixing an optically active clausenamide derivatives of formula (II) with pharmaceutically acceptable diluents or excipients.

Furthermore, the pharmaceutical preparations may be various administration form such as a sterile isotonic aqueous solution, tablet, capsule, pill, drop and suppositories, paster, oilment, gel, pastes, cream, spray (including aerosols), lotion, suspensions, solution and emulsions of the active ingredient in aqueous or non-aqueous diluents, syrups, granules or powders.

The diluents to be used in pharmaceutical compositions (e.g. granulates) include customary pharmaceutically acceptable diluents or excipients such as starch, gelatine, silicic acid, polyethylene glycol. For liquid pharmaceutical preparation, a diluent or carrier may be water, alcohol, proplene glycol, vegetable oil, corn oil, peanut oil, olive oil, surface active agents, lubricants, disintegrating agents, preservative agent, flavoring agent or pigment.

According to the present invention, the term "patient" means to mammal, for example, human being.

The term "neurodegenerative disease" includes, for example, cerebral ischemia, Alzheimer disease and vascular dementia etc.

The levoclausenamide [(-)3S, 4R, 5R, 6S]] as one of some optically active clausenamide derivatives (not forming part of the present invention) may be prepared by the de novo asymmetic synthesis and the said detailed synthesis is shown in the following scheme 1.

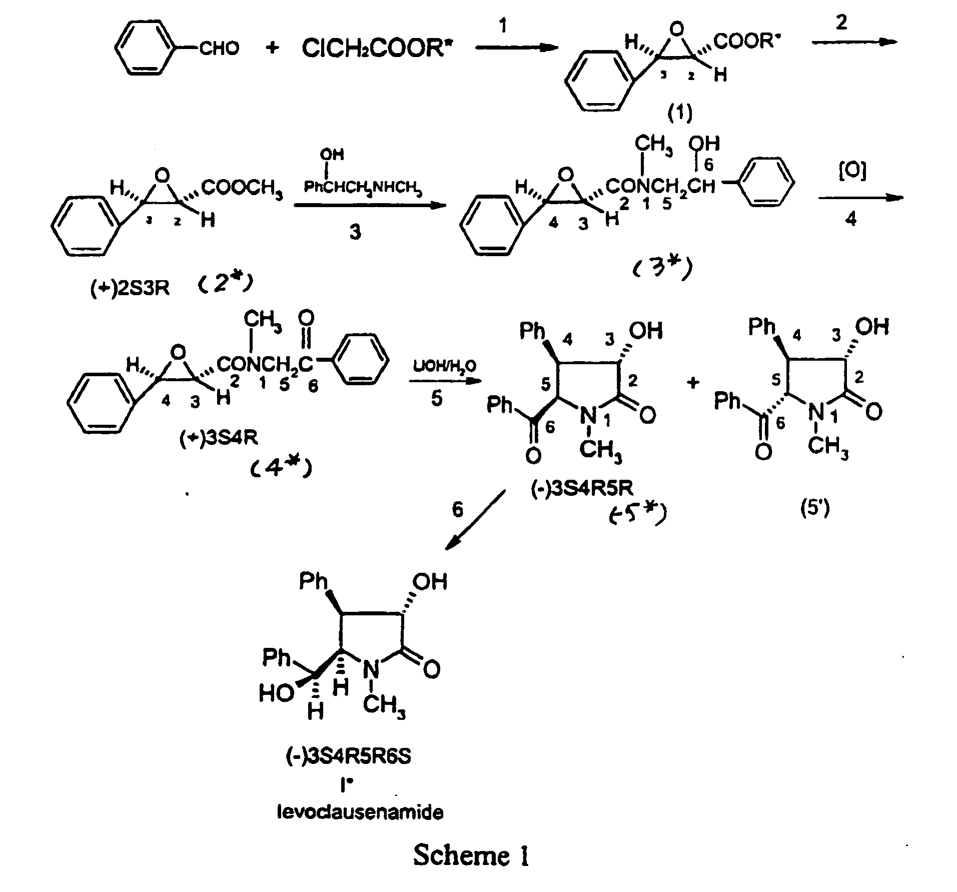

Scheme 1 consists of the following steps:
i) treating benzaldehyde with optical alkyl (R) chloroacetate in the presence of basic catalyst to give compound(1),the chiral group from natural or synthetic alcohol with appropriate configuration,preferably(+) menthol, (+) 8-phenylmenthol,(+) 8- β - naphthyl menthol,
ii) adding sodium methoxide to a solution of compound(1) in methanol to yield compound(2*),(+)2S3R methyl 2,3 epoxy cinnamate,
iii) interchanging easter group of compound(2*) with β -phenyl-ethanol-N-methyl amine in the presence of basic catalyst to yield compound (3*), 3S,4R-N-methyl-N-(β-hydroxyl-ethylbenzyl) 2,3 epoxy cinnamate compound(3*), or
   said compound (3*) is obtained directly with compound 1 by the procedure as described in (iii),
iv) oxidizing compound 3 with potasssium permanganate and cuppric sulfate to afford (+) 3S4R-N-methyl-N-benzoylmethyl-2,3 epoxy cinnamide(4*)
v) treating the above compound 4 with basic catalyst to give the cyclized product (-) clausenamidone(5*) ,base catalyst may be selected from lithium hydroxide, sodium hydroxide, potassium hydride, lithium diisopropyl amide, butyl lithium, tetraalkyl ammonium hydroxide or trialkyl amine. The solvent in the reaction may be selected from water, menthnol, ethanol, low molecular alkanol, or aqueous alkanol.
vi) reducing compound(5*) with boronhyde class of reducing agent such as lithium boronhydride, sodium boronhydride, lithium tris sec-butyl boron hydride to give (-) 3S, 4R, 5R, 6S levoclausenamide I*.
Note: sign* meaning optically active, R is alkyl.

The Levoclausemamide (prepared by scheme 1) may also be prepared by biocatalytic resolution of the starting material racemic methyl 2,3 epoxy cinnamate with hydrolase producing microorganisium to give (+) 2S3R-methyl 2,3 epoxy cinnamate in scheme 2, then repeating the steps iii)-vi) in Scheme 1, thereby obtaining levoclausenamide.

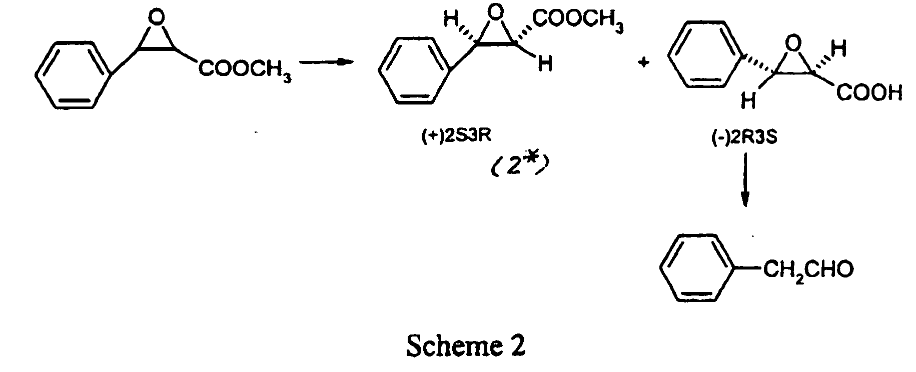

In scheme 2, The hydrolase producing microorganisium used may be selected from such as fungi(e.g. Aspergillus, sp; Mucor sp; Penicillium, sp;Phizopus, sp), bacteria (e.g. Achromobacter, sp; Alcaligenes, sp; Bacillus, sp; Brevibacterium, sp; Corynebac- terium, sp; Erwinia, sp; Pseudomonas, sp), yeast (e.g.Canadida, sp;Pichia, sp), Rhodotorula, sp; Hansenula, sp) or Actomycetals (e.g. Nocardia,sp;Strep- tomyces,sp). The above mentioned microorganisium may be cultured under acrobic condition in medium containing carbon, nitrogen source with appropriate inducer and inorganic salt at room temperature or heating, preferably at 20-40°C, PH5-8,the mycelium produced is collected for stereo-selective hydrolysis of racemic methyl 2,3 epoxy cinnamate as shown in scheme 2.

To the above mentioned culture medium surface active agent such as polyethylene glycol, tween, polyvinyl alcohol, hexadecyl-trimethyl ammonium bromide at concentration of 0.5-5%, preferably 0.5-2% may be added, if necessary.

The stereoselective hydrolysis is proceeded in appropriate organic solvent such as benzene, toluene, xylene, ethyl-ether, ethyl acetate, isopropyl ether, carbon tetrachloride, chloroform preferably toluene, xylene or isopropyl ether, and in such condition that the concentration of substrate as 0.05-10%, preferably 0.5-5%, 10-50°C of temperature, PH 5-10, preferably 6-9.

Furthermore, the above mentioned enzymatic hydrolysis is carried out in aqueous buffer solution or in both of biphasic aqueous buffer and organic solvent such as toluene, benzene, ether, ethyl acetate, isopropyl ether, toluene, carbon tetrachloride or chloroform, preferable toluene, xylene, or isopyl ether.

The isolation of the optical active methyl 2,3 epoxy cinnamate as product is conducted by the known process in the art, such as, extracting the reaction mixture with organic solvent or separating the organic layer in case of biphasic reaction being applied, washing the organic extract or organic layer with saturated sodium bisulfite solution to remove the phenylacetaldehyde, drying, concentrating to give compound 2, (+) 2S3R mehyl 2,3 epoxycinnamate.

The levoclausenamide (prepared by scheme 1 or scheme 2) may also be prepared by resolution of the intermediate, racemic clausenamidone. The details is shown in scheme 3.

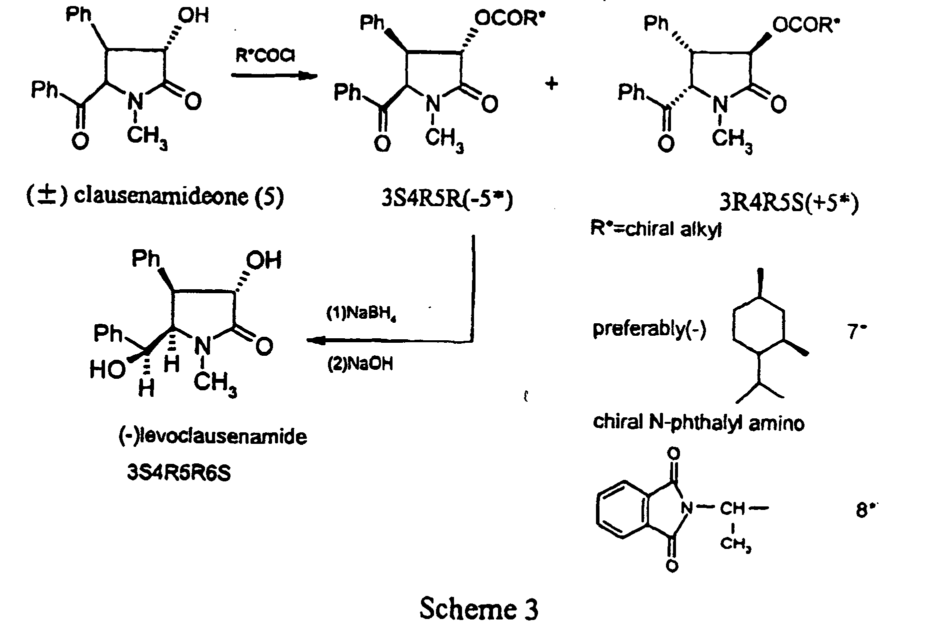

In scheme 3, the resolving agent is selected from chiral acid chloride such as optically active alkyoxy substituted acylhalide, N-phthalyl optical active amino acid halide, or optical active sulfonyl halide, preferably menthoxy active chloride, N-phthalyl (-) alanyl chloride.
said resolution comprising following steps:
i') easterifying racemic clausenamidone with optical active acyl chloride in the presence of pyridine,
ii') separating the distereomers (-5*)(+5*) by recrystallization or column chromatography.
iii') reducing (-5*) by sodium borohydride followed by hydrolysis in the presence of NaOH, giving levoclausenamide (-I*)

The present invention is further directed to the method of preparation of optically active clausenamide derivatives of the general formula II selected from one of the following compounds. 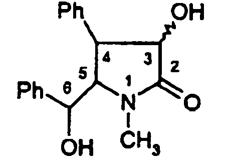 3S4R5R6R (-) epiclauseanamide,
3R4S5S6S (+) epiclauseanamide,
3S4R5S6S (-) epineoclauseanamide
3R4S5R6R (+) epineoclauseanamide
3S4S5S6R (+) cisclauseanamide,
3R4R5R6S (-) cisclauseanamide,
3S4S5S6S (+) cisepiclauseanamide,
3R4R5R6R (-) cisepiclauseanamide,
3S4S5R6S (-) cisneoclauseanamide,
3R4R5S6R (+) cisneoclauseanamide,
3S4S5R6R (-) cisepineoclauseanamide and
3R4R5S6S (+) cisepineoclauseanamide,
wherein the process may be summarized in following Scheme 4 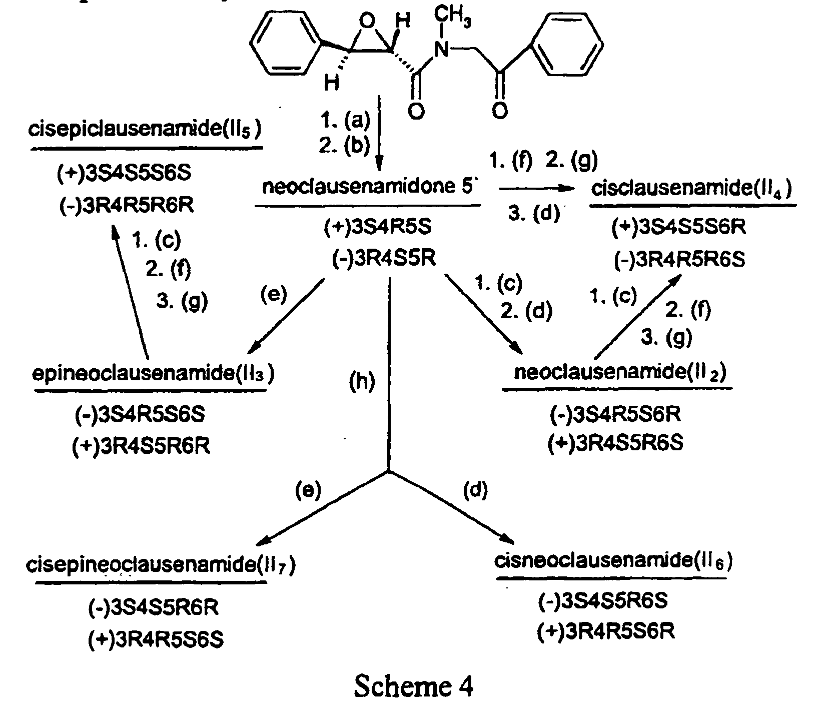 Note:
(a) base catalyzed cyclization, NaOCH₃ or (CH₃)₄NOH
(b) resolution, resolving agent (-)menthyoxyl acetyl chloride
(c) derivation of C₃-OH by dihydropyan or acetylchloride/pyridine
(d) reduction of C₆=O by reducing agent NaBH₄ or L-selectride
(e) reduction of C₆=O by reducing agent Al(i-C₃H₇O)₃
(f)oxidation of C₃-OH by oxidizing agent, K₂Cr₂O₇/H⁺, or KmnO₄+CuSO₄, MnO₂ or DMSO/(COCl)₂
(g) reduction of ^{2, 3} by NaBH₄/H⁺, or NaBH₄/AlCl₃, or catalytic hydrogenation
(h) invertion of C₃-OH by DIAD or DEAD/TPP, H⁺(Mitsunobu method) or by CsOAc/18-crown-8
or the process for preparation of epiclausenamide is showed in scheme 5: 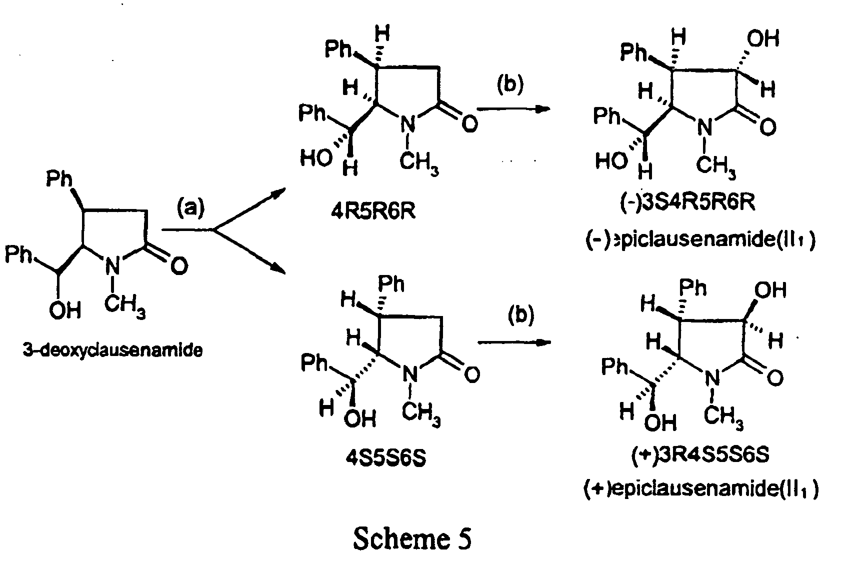 Note:
(a) resolution of racemic 3-deoxyepiclausenamide by chiral optical active acid such as (-) menthyloxy acetic acid, N-phthalyl s-alamine, O-acetyl-mandelic acid,
(b)hydroxylation of C₃ by LDA/P(OEt)₃/O₂
(c) starting material racemic 3-deoxyepiclausenamide is known and obtained by the known method in the art.

According to the present invention, the following optically active clausenamide derivatives having following physico-chemical features have been obtained.

| Compound | Absolute configuration | mp°C | (α)^{L}_{D} (c, solvent) |
|---|---|---|---|
| Reference (-)clausenamide | 3S, 4R, 5R, 6S | 161-162 | -146(0.21, MeOH) |
| Reference (+)neoclausenamide | 3R, 4S, 5R, 6S | 186-187 | +89.5 (0.2, MeOH) |
| Reference (-)neoclausenamide | 3S, 4R, 5S, 6R | 186-187 | -88.6 (0.18, MeOH) |
| (+)cpiclausenamide | 3R, 4S, 5S, 6S | 108-110 | +201 (0.245, MeOH) |
| (-)epiclausenamide | 3S, 4R, 5R, 6R | 107-109 | -204 (0.445, MeOH) |
| (+)cpineoclausenamide | 3R, 4S, 5R, 6R | 220-222 | +36.5 (0.15, MeOH) |
| (-)epincoclausenamide | 3S, 4R, 5S, 6S | 221-222 | -37.7 (0.16, MeOH) |
| (+)cisclausenamide | 3S, 4S, 5S, 6R | 197-199 | +6.30 (0.46, CHCl₃) |
| (-)cisclausenamide | 3R, 4R, 5R, 6S | 196-198 | -6.07 (0.675, CHCl₃) |
| (+)cisepiclasuenamide | 3S, 4S, 5S, 6S | 198-199 | +39.7 (0.785, CHCl₃) |
| (-)ciscpiclausenamide | 3R, 4R, 5R, 6R | 199-202 | -38.37 (0.66, CHCl₃) |
| (+)cisneoclausenamide | 3R, 4R, 5S, 6R | 164-166 | +66.7 (0.46, MeOH) |
| (-)cisneocluasenamide | 3S, 4S, 5R, 6S | 168-170 | -65.3 (0.32, MeOH) |
| (+)cisepineoclausenamide | 3R, 4R, 5R, 6S | 275-277 | +31.2 (0.31, DMSO) |
| (-))cisepineoclausenamide | 3S, 4S, 5R, 6R | 271-273 | -33.3 (0.34, DMSO) |

The present invention is further directed to the facilitating learning and memory activity and inducing long-term potentiation of some optically active clausenamide derivatives. Their nootropic action was 5-10 times and 50-100 times more than that of (±)-clausenamide and piracetam respectively. For example, (-)clausenamide could also improve spatial memory impairment induced by β -amyloid (Aβ 25-35). The nootropic mechanisms may be deduced as follows:
1. (-) clausenamide was a potassium channel blocker which induces an increase of intracellular Ca²⁺ level and then actiate cAMP and PKC to facilitate memory and LTP.
2. (-) clausenamide increased synapses density in weaning mice and mossay fiber sprouting in adult rat hippocampus.
3. (-) clausenamide increased Ach content and ChAT activity in cortex, hippocampus and striatum.
4. (-) clausenamide increased protein biosyntheses of mice brain and promoted zif/268 mRNA and protein expression.

Some of optically active clausenamide derivatives of the invention unexpectedly shows the improvement of hydpoxia, nootropic, neurodegerrerative diseases such as cerebral ischemia, Alzheimer diseases, vascular and antiazonit action of neurocell apoptosis. In particular, for example, (-) clausenamide at concentration of 10⁻⁷-10⁻⁵ mol/l inhibited apoptosis significantly In three apoptotic models,. Further studies indicates that the inhibitory of (-) clausenamide on apoptosis may be relevant with its activity in respect of promoting Bcl-2 expression, raising the mitochondrial complex I and complex IV activity and inhibitory release of cytochrome C.

### Description of Drawings

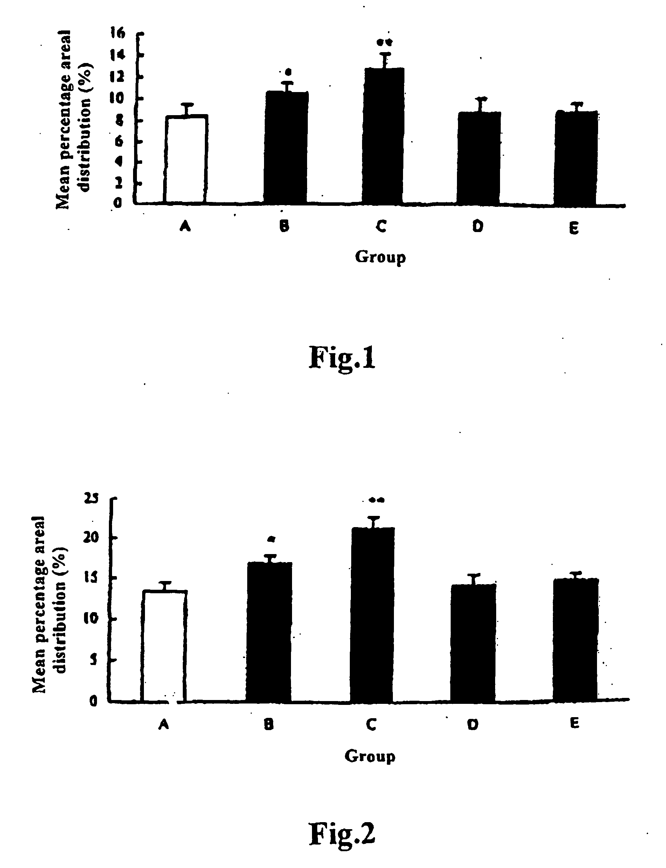

Figure 1 represents that areal distribution of mossy fiber sprouting in the fascia dentata of rats from control group (A), (-)-Clausenamide (8 mg/kg) group (B), (-)-Clausenamide (40mg/kg) group (C), (+)-Clausenamide (8 mg/kg) group (D), (+)-Clausenamide (40 mg/kg) group (E). *P<0.05, **P<0.01 vs control group.
Figure 2 represents that areal distribution of mossy fiber sprouting in the hippocampal CA3 region of rats from control group (A), (-)-Clausenamide (8 mg/kg) group (B), (-)-Clausenamide (40mg/kg) group (C), (+)-Clausenamide (8 mg/kg) group (D), (+)-Clausenamide (40 mg/kg) group (E). *P<0.05, **P<0.01 vs control group.

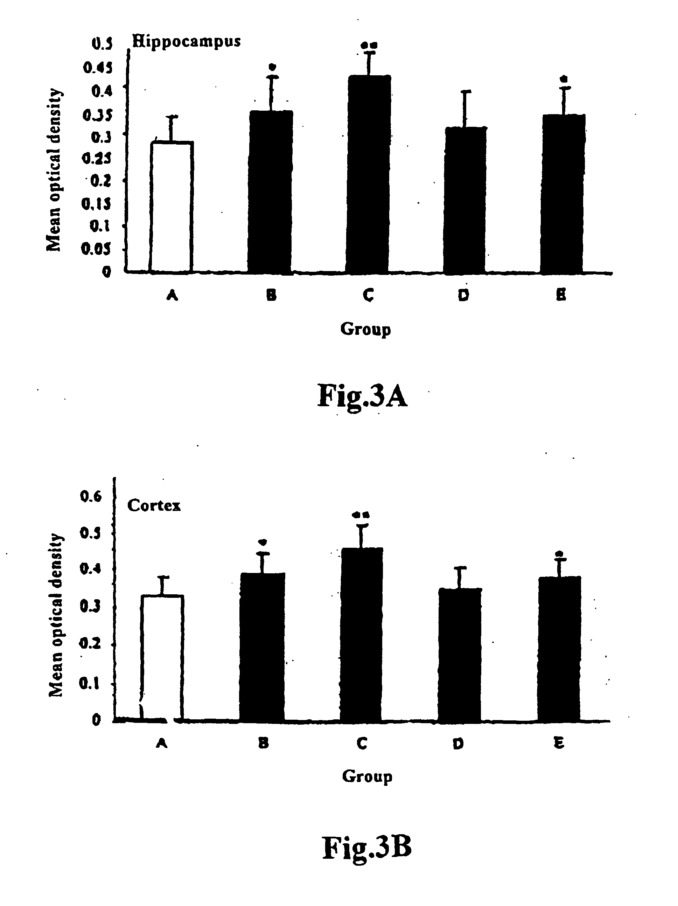

Figure 3A and Figure 3B represent that Relative amount of BDNF protein expressed in the hippocampal pyramid cells and parietal cortical neurons of rats from control group (A), (-)-Clausenamide (8 mg/kg) group (B), (-)-Clausenamide (40 mg/kg) group (C), (+)-Clausenamide (8 mg/kg) group (D), (+)-Clausenamide (40 mg/kg) group (E). *P<0.05, **P<0.01 vs control group.

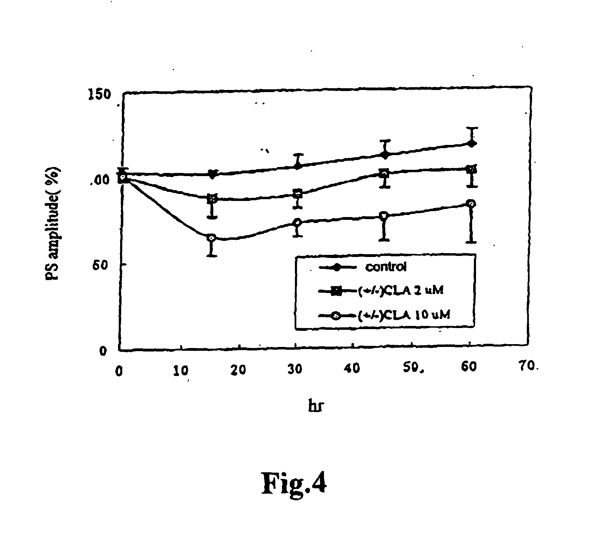

Figure 4 represent effects of(±) clausenamide at concentration of 2µM and 10µM on PS amplitude (%)

The following examples 1-17 are reference examples.

### Example 1: Determination of the absolute configuration of levoclausenamide

(-)Clausenamidonyl (-)menthy oxyacctate (-7*) m.p.177-178°C, [α] ¹_{D}⁸ =-249(c 0.21,CHCl₃) was prepared and its single crystal was analyzed by x-ray diffraction. From the x-ray diffraction pattern of the ester(-7*) the configuration of (-) clausenamidone was identified as 3S, 4R, 5R based on the known configuration of the (-) menthyl in the molecular. After hydrolysis of the ester with acid catalyst, gave the (-) clausenamidone,m.p. 191.5-193.5°C, [α] ¹_{D}⁸ =-140.9°(c=0.3,CH₃OH)

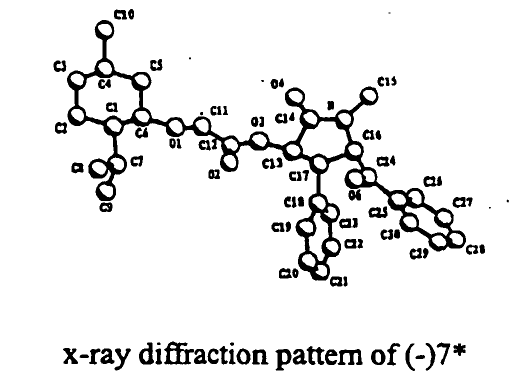

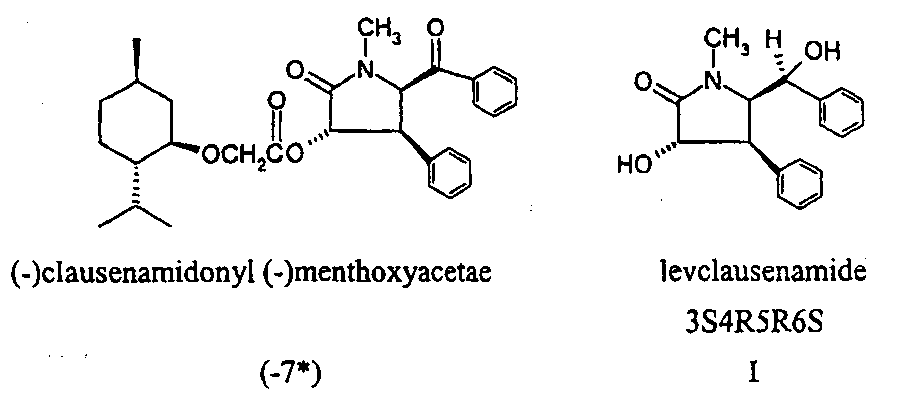

### Examples 2-8 illustrate synthesis of levoclausenamide as described in scheme 1.

### Example 2: Preparation of (+)8-β-naphthyl menthyl (+) 2S3R,2,3 epoxy cinnamate(2a*)

Sodium hydride (80%) 430mg was added to 20ml dry THF. The solution was gradually dropped to a solution of 1g benzaldehyde and 2.3g of (+) 8-β -naphthyl chloroacctate in 10ml of THF. After the reaction mixture being stirred about 40min at room temperature, it was poured into 30ml ice water containing 1ml of acetic acid and then extracted with ether. The ether extract was washed with saturated NaHSO₃ solution followed by saturated NaHCO₃ and NaCI solution. It was dried and concentrated to give 2.9g oily residue. White solid of 1.16g as title compound was obtained after chromatograph on silica gel column eluted with petroleum ether/ethyl acetate (10:1), yield 79.0%,m.p.38-40°C,[ α ] ¹_{D}⁵ =+35.8°(c 1.31, CHCl₃).
Note: NaOCH₃ also can be used .

### Example 3: Preparation of (+)2S3R menthyl 2,3 epoxycinnamate(2b*)

A solution of 3.27g of NaH (80%) in 100ml anhydrous THF was added dropwise to 11.6g benzaldehyde and 11.85g of (+) menthyl chloroacetate in 100ml dry THF. Afterward it was stirred at room temperature for 10 hrs. Then it was filtered and filtrate was evaporated. To the residue ether was added. The ether solution was washed successively with saturated NaHSO₃ aqueous solution, NaHCO₃ solution and NaCl saturated solution until neutral. Drying with Na₂SO₄, filtered and evaporated to give oily residue which solidified on standing. After recrystallized three times from ethanol yielded 7.62g (35% yield) of white crystalline solid as title compound, m.p. 62-63°C,[α] ¹_{D}⁵ =+167°(c 1.0,CHCl₃).

### Example 4: Preparation of(+) 2S3R N-methyl 2,3 epoxy cinnamate (2*)

To a solution of 4.3g compound 2*a or 2*b of example 2 and 3 in 80ml methanol 200mg of NaOCH₃ was added with stirring. After 6 hrs,0.2ml HOAc was added, the reaction mixture was concentrated under reduced pressure. The residue was stirred with 100ml ether for 30min. Filtring and ether was removed by evaporation. The 4.3g residue was chromatographed on silica gel eluted with petroleum ether/ethyl acetate (20:1) to give 1.33g oily product(2*) as title compound, 75% yield. [α] ¹_{D}⁵ =+170.9°(c 1.33,CHCl₃).

### Example 5: Preparation of (+)2S3R N-methyl, N-(β -benzylethyl)-2,3 epoxycinnamate(3*)

A solution of 1.78g compound (2*) of example 4 in 5ml of methanol and a solution of 1.51g N-methyl-N-(β-benzyl) ethanol amine in 5ml methanol was cooled separately to -20°C and then mixed. To the mixture 100mg NaOCH₃ was added. The reaction mixture was kept in the refrigerator for two days. 0.2ml of 2N HCl was then added. After removal of the solvent by evaporation 2.3g of compound (3*) was obtained, yield 77.3%,m.p. 93-93°C, [α]¹_{D}⁵ =+70.2°(c 0.68,CH₃OH).

### Example 6: Preparation of (+) 2S3R N-methyl-N-(benzoyl methyl) 2,3 epoxy cinnamate (4*)

To a solution of 2.97g compound (3*) in 100ml of CH₂Cl₂,was added 2.2g of CuSO₄ and 6.32g of KMnO₄.The suspension was stirred at room temperature for 3hrs.The reaction mixture was filtered and the filter cake was washed thoroughly with CH₂Cl₂. The combined filtrate and washing solution was concentrated to yield 2.9g of oily residue. After crystallization from ethyl acetate/petroleum ether (1:1), 2.3g(78% yield) of compound (4*) was obtained, m.p. 87°C, [ α ] ¹_{D}⁵ =+140.2°(c 0.5,CH₃OH).

### Example 7: Preparation (-) 3S4R5R clausenamidone (5*)

A solution of 6.6mg LiOH H2O in 30ml aqueous methanol was stirred with 2.95g of compound (4*) at 35°C for 8hrs.It was cooled and filtered to give compound (5*) which after recrystallization from methanol ethyl acetate yielded 1.5g (5*)(51% yield) (-) clausenamidone m.p. 196-198°C, [α]¹_{D}⁵ =-338°(c 0.6,CH₃OH)

### Example 8: Preparation of levoclausenamide(I*)

To a solution of 2.95g of compound (5*) ,(-) clausenamidone,1.52g of NaBH₄ was added while stirring. Afterwards stirring was continued for 3 hrs. Thereafter 1ml of 2NHCl was added. Then the solvent is removed by evaporation and the residue left behind was dissolved in CH₂Cl₂.Filtered and concentrated to afford 2.4g of compound (I*) levoclausenamide yield 81%,m.p. 160-161°C, [α]¹_{D}⁵ =-144°(c=0.2,CH₃OH).

### Example 9-13 illustrate the biocatalytic resolution of the starting material racemic methyl 2,3 epoxycinnamate to (+) 2S3R methyl 2,3 epoxy cinnamate.

### Example 9: Resolution of racemic methyl 2,3 epoxycinnamate by fungi (aspergillus sp) enzyme

Thirty ml of culture medium of 3% suger,1% peptone, 0.5% potassium dihydrogen phasphate, 0.5% polyethylene glycol,1% olive oil in 250ml flask was sterilized at 120°C under 1 atm. After cooling it was inoculated with Aspergillus,sp and cultured for 40hrs at about temperature 30°C and with 220 rpm. The mycelilium was collected by centrifegalization and was added to 10ml phosphate buffer solution (pH7) containing 150mg of (±) methyl 2,3 epoxy cinnamate and the obtained reaction mixture was kept at about 30°C for 72hrs.At the end of reaction , the reaction mixture was extracted twice with ethyl acetate(20ml). The extract was washed with saturated solution of NaHCO₃ followed by saturated solution of NaCl, dried with MgSO₄ and filtered. The solvent was removed by evaporation. An oily residue 6.6mg was obtained. It was analyzed by chiral gas chromatograph, which shows ee% 98, [ α ] ²_{D}⁰ =+180°(c=1,CHCl₃) for (+) 2S3R methyl 2,3 epoxycinnamate.

### Example 10: Resolution of racemic methyl 2,3 epoxycinnamate by yeast Canadida sp enzyme

Thirty milliliters culture medium of 4% surger,2% peptone, 1% poassium dihydrogen phosphate, 2% polyethylene glycol, 1% olive oil in 250ml flask was sterilized at 120°C and under 1 atm for thirty min. It was inoculated with cyclic yeast Candida sp and kept at 30°C, with 200 rpm for 40hrs.The mycilium was separated and added to 300mg of racemic methyl 2,3 epoxycinnamate in 30ml of phosphate buffer solution (pH8). After 72 hrs at 30°C,the reaction mixture was treated as described in example 9 to give 120mg of (+) 2S3R methyl 2,3 epoxycinnamate with ee%98, [α] ²_{D}⁰ =+180°(c=1,CHCl₃).

### Example 11: Resolution of racemic methyl 2,3 epoxycinnamate by bateria Achromobacter sp enzyme

Thirty millilitres culture medium containing 2.5% sodium citrate,1% soybean peptone,0.5% potassium dihydrogen phosphate,2.5% polyethylene glycol,1% olive oil was sterilized at 120°C and under 1 atm for 30min.The culture medium was inoculated with cyclic Achromobacter sp. and incubated at 40°C with 299 rpm for 40hrs.The mycilium was separated and added to 19ml phosphate buffer solution (pH7) containing 150mg of racemic methyl 2,3 epoxycinnamate.After 72hrs at 30°C the reaction mixture was treated and analyzed as described in example 9 to yield 66mg of oily substance,(+)2S3R methyl 2,3 epoxycinnamate with ee%99, [α]²_{D}⁰ =+181°(c=1,CHCl₃).

### Example 12: Resolution of racemic methyl 2,3 epoxycinnamate by bacteria,Bucillus sp enzyme

Thirty millilitres of culture medium containing 20% of lactic acid,1.5% ammonium sulfate,0.5% potassium dihydrogen phosphate,0.5% polyethylene glycol,0.1% olive oil were sterilized at 120°C and under 1 atm for 30min.The culture medium was inoculated with Bucillus sp and incubated at 50°C with 220 rpm for 40hrs.The mycilium was collected and added to 150mg of racemic methyl 2,3 epoxycinnamate in 10ml phasphate buffer solution (pH=10).The reaction was carried out at 50°C for 72hrs. And then treated and analyzed as described in example 9.66 mg of oily product (+) 2S3R methyl 2,3 epoxycinnamate was obtained as ee%99, [ α ] ²_{D}⁰ =+181°(c=1,CHCl₃).

### Example 13: Resolution of racemia methyl 2,3 epoxycinnamate by Actomycetales Nocardia sp. Enzyme

The 30ml of culture medium containing 1% of surgar,20% peptone,0.3% potassium dihydrogen phasphate,1% polyethylene glycol,2% olive oil in 250ml flask was sterilized at 120°C and under 1 atm for 30min.After cooling, it was inoculated with cyclic Nocardia sp. and incubated at 30°C with 250rpm for 36hrs.The mycilium was collected and added to 220mg of racemic methyl 2,3 epoxycinnamate in 10ml buffer solution (pH7.5). The reaction was carried out at 30°C for 72hrs.At the end of reaction, the reaction mixture was treated and analyzed as described in Example 9. 80 mg of oily (-) 2S3R methyl 2,3 epoxycinnamate was obtained,ee%98, [ α ] ²_{D}⁰ =+180°(c=1,CHCl₃).

### Examples 14 and 15 illustrate the preparation of levoclausenamide by resolution of intermediate racemic intermediate clausenamide (Scheme 3)

### Example 14: Preparation of levoclausenamidone through resolution of racemic clausenamidone(Scheme 4)

The mixture of 3g menthyoxylacetic acid in 10g SOCl₂ was refluxed for 5hrs and then SOCl₂ was removed. Five milliliters of toluene was added and evaporated at reduced pressure to drive the residual SOCl₂, 3.5g brownish residue was dissolved in 50ml CH₂Cl₂ and to which 2g racemic clausenamidone(±5) was added. Pyridine lml was added while cooling, and stirred 4hrs at room temperature. Two spots were showed by TLC. The reaction mixture was washed successively with 2N HCl, saturated NaHCO₃ aqueous solution, saturated NaCl aqueous solution and dried over Na₂SO₄. After removing the solvent 5.4g of bromnish solid was obtained. After first recrystallization from methanol to give -7*, the mother liquid was chromatographed on silica gel column eluted with ethyl ether/hexane (2:1) to give +7* and more -7*.
(-7*): 1.06g,yield 30.9%(theoretical yield 50%),m.p.:177-178°C, [ α ] ¹_{D}⁸ =-249°(c 0.21,CHCl₃).
(+7*): 0.86g, yield 24.8%(theoretical yield 56%),m.p.:152-153°C, [ α ]²_{D}⁰ =+156°(c 11,CHCl₃).
(-7*) and (+7*) were hydrolyzed separately in 30ml methanol catalyzed by PTS. After refluxing for 2hrs. Methanol was taken off by evaporation and the residue was dissolved in CH₂Cl₂.The CH₂Cl₂ solution was first washed with acid and followed by base and water.(-)clausenamidone(-5*) and (+) clausenamidone(+5*) was obtained respectively from (-7*) and (+7*) after removal of solvent.
(-)clausenamidone(+5) yield 88%, m.p.:193-194 °C , [ α ]¹_{D}⁸ =-340°(c=0.22,CH₃OH).
(+)clausenamidone(-5) yield 82%, m.p.:193-194 °C , [ α ] ¹_{D}⁸ =+339°(c=0.21,CH₃OH).
(-)clausenamidone(-5*) 0.59g was reduced as described in Example 8 with 0.11g NaBH₄.Recrystallization from methanol of the product gave 0.5g of levoclausenamide, yield 83%, m.p.:162-163 °C , [ α ] ¹_{D}⁸ =-145°(c=0.24,CH₃OH).

### Example 15: Resolution of racemic clausenamidone (Scheme 5)

A solution of 3.56g N-phthayl (-) L-alanine and 5g of SOCl₂ in 50ml toluene was refluxed for 6hrs.Toluene and CH₂Cl₂ were removed under reduced pressure. To the renction mixture 20ml toluene was added again and evaporated to drive the residual SOCl₂.Then 100ml of SOCl₂ was added and cooled to <0°C.

To above solution racemic clausenamidone 5g was added under cooling and stirring and followed by adding 1.6g pyridine dropwise. The reaction mixture was stirred for 10hrs at room temperature. After 30ml water was added and the organic layer was separated. The aqueous layer was extracted with 30ml CH₂Cl₂.The extract and organic layer was combined and washed with 30ml of 2N HCl,saturated NaHCO₃ aqueous solution and NaCl aqueous solution. Dried over Na₂SO₄ and filtered. The solvent was removed. The residue was recrystallized with ethyl acetate to give cubic crystalline product 1.42g yield 21%(theoretical yield 56%),m.p.:189-190°C, [α] ¹_{D}⁵ =-241°(c 0.5,CHCl₃)(-7).
The crystal was dissolved in 200ml methanol, then reduced with 1.52g NaBH₄ under stirring for 5hrs at room temperature. After 1ml 2NHCl was added and concentrated under reduced pressure. The residue was recrystallized to give 2.38g of levoclausenamide, yield 81%,m.p.:161-163°C, [α] ²_{D}⁰ =-144.4°(c=0.46,CH₃OH).

### Examples 16-19 illustrated the method concerning the preparation of some new optically active clausenamide derivative (Scheme 4 and 5) but not limited

### Example 16: Resolution of racemic neoclausenamidone 5'

(±)Neoclausenamidone 2.66g was added to 30ml CH₂Cl₂ solution of menthyoxy acetyl chloride prepared from 2.71g of the acid. The solution was cooled by ice-water and 1.5ml of pyridine was added. Stirred at room temperature for Shrs.Then the reaction mixture was dilute with 50ml CH₂Cl₂ and was washed with 2N HCl,saturated NaHCO₃ solution,50ml NaCI solution successively. Dried with Na₂SO₄ and concentrated to give 6g of oily product which is solidified by adding 250ml of hexane. The solid was filtered and recrystallized with methanol to give 1.49g of C₃-ester(a), m.p.:170-172°C, [α]¹_{D}⁵ =-50.9°(c 1.25,CHCl₃) ,yield 34%(theoretical yield 50%) .The hexane and methanol filtrate was chromatographed on silica gel column to give C₃-ester (b), m.p.:104-105°C, [α]¹_{D}⁵ =-31.2(c 1.4,CHCl₃) ,yield 40%. The above ester (a) and ester(b) were hydrolyzed as described in example 14 From 6g of ester (a) gave 3.25g , 86% yield of (-) neoclausenamidone(-5'*)(3R4S5R). m.p.:164-167 °C , [α] ¹_{D}⁵ =14.9°(c 0.6,CHCl₃)
From 5.5g of ester (b) gave 3.0g, 89% yield of (+) neoclausenamidone(+5'*)(3S4R5S). m.p.:167-169 °C, [ α ] ¹_{D}⁵ =+14.1°(c 0.56,CHCl₃)

### Example 17: Preparation of optical active cisclausenamide from optical active neoclausenamidone(5'*)

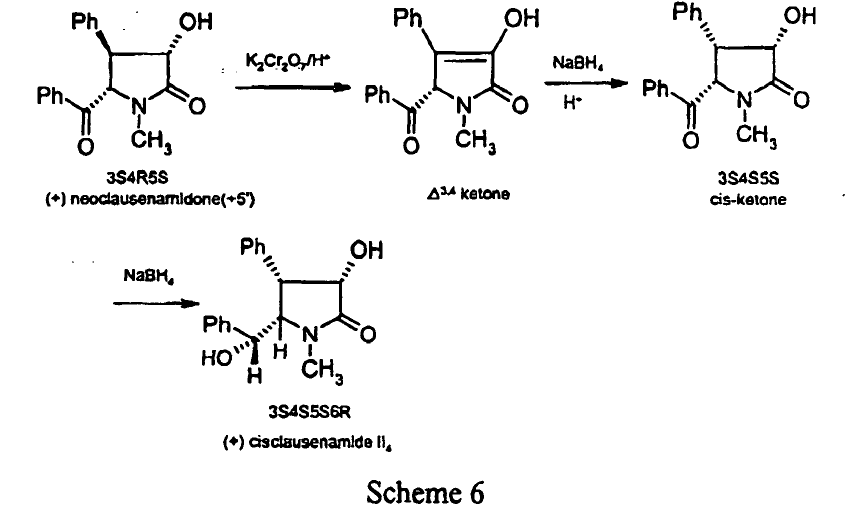

An acidic K₂Cr₂O₇ solution made from 0.66g K₂Cr₂O₇ and 0.66g concentrate H₂SO₄ and 5ml of water was added to a solution 0.25g of (+) neoclausenamidone in 10ml CH₂Cl₂ with stirring at 25-30°C.After stirring for about 5hrs. 20ml of CH₂Cl₂ was added. The organic layer was separated and washed with a saturated NaHCO₃ solution followed by water. The solvent was taken off, the obtained solid was recrystallized with methanol to give 0.19g(yield 78%) of Δ^{3,4} neoclausenamidone,m.p. 157-159°C,[α]¹_{D}⁵ =-199.6°(c 0.75,CHCl₃).

The above product 0.10g was dissolved in 10ml CH₂Cl₂ containing 1ml acetic acid, 0.01g of NaBH₄ was added by fraction with stirring. The reaction was monitored by TLC. At the end of reaction, acetic acid dropwise to react with excess NaBH₄ left. Then 20ml CH₂Cl₂ was added and the reaction mixture was poured in to 50ml of ice water. The organic layer was separated and washed with a saturated NaHCO₃ solution and water. The solution was evaporated and the residue was chromatographed on silica gel to give 0.074g, yield 73% of cis-ketone, m.p. 145-147°C,[ α ] ²_{D}⁰ =+111.0°(c 0.48,CHCl₃).

The cis-ketone 0.08g was dissolved in 10ml CH₂Cl₂ and reduced with 0.01g of NaBH₄/CH₃OH.At the end of reaction 30ml of CH₂Cl₂ was added followed by acetic acid to react with excess NaBH₄.The organic layer was washed with a saturated solution of NaHCO₃ and then water. The solvent was evaporated to leave an oily residue. It was crystallized with acetone and petroleum ether, and the crystalline of solid (+) 3S4S5S6R cisclausenamide 0.59g (77%) with m.p. 197-199.5°C,[α]²_{D}² =+6.30°(c 0.46,CHCl₃) was obtained.

The above procedure was applied to the preparation of (-) 3R4R5S6R cisclausenamide from (-) neoclausenamidone(-5').The corresponding Δ ^{3,4} clausenamidone obtained yield 75%,m.p. 154-156°C,[ α ] ¹_{D}⁵ =+206.8°(c 0.90,CHCl₃),cis ketone 3R4R5R yield 76%, m.p. 130-132°C,[ α ] ²_{D}⁰ =+116.5°(c 0.40,CHCl₃),(-)cisclausenamide 3R4R5R6S yield 69%, m.p. 196-198°C, [ α ] ²_{D}² =-6.07°(c 0.67,CHCl₃)

### Example 18: Preparation of optical active epiclausenamide II₁(see Scheme 5)

To a solution of 60 mg 3-deoxyepiclausenamide in 30ml CH₂Cl₂ was added 621mg of O-acctylmandelic acid, 26mg of 4-dimethyl-aminopyridine and 880mg of 1,3 dicyclohexyl carbodimide. The reaction mixture was stirred 1hr at room temperature. It was filtered and the filler cake was washed successively with 5ml of 2N HCl,saturated solution of NaHCO₃ and NaCl solution. After dried with Na₂SO₄,the solvent was taking off by evaporation . The oily residue was chromatographyed on silica gel column eluted with ethyl acetate/petroleum ether (1:2) and recrystallized from ethyl acetate to give two products: A, m.p.:212-215°C, yield 46%.B, m.p.:206-209°C, yield 47%.A and B were hydrolyzed separately in mixtures of CH₃OH and 10ml CH₂Cl₂ in the presence of 141mg of K₂CO₃ at room temperature for 12hrs.The reaction mixtures were treated as usual. From A(yield 93%) of (+) 3-deoxyepiclausenamide m.p.:239-242°C,[α]³_{D}⁰ =+137(c 0.46,CH₃OH) and from B(yield 90%) m.p.:237-240°C,[α]³_{D}⁰ =-136(c 0.48,CH₃OH)

The optical active 3-deoxyepiclausenamide(120mg) was dissolved in a mixture of 5ml THF and 1.3ml HMP. The solution was cooled -70°C,and stirred for 5min,and then 1.40ml LDA((C₂H₅)₃NLi) was added and stirred 1hr at -60 to -70°C under nitrogen. The above solution turned red, and 53µl P(OEt)₃ were added. The reaction was taken place under a stream of dried oxyen at -60 to -70°C for 2hrs.At the end of reaction, the reaction mixture were adjusted to pH3-4 with 0.5N HCl in an ice bath. The reaction mixture were extracted with ethyl acetate four times,20ml each time. The organic layer then washed with saturated NaCl solution and dried with Na₂SO₄.The solvent was driven off by evaporation to give an oily residue which was chromatographed on silica gel column eluted with ethyl acetate/petroleum ether (1:3) followed by recrystallization with ethyl acetate. Optical active epiclausenamide was obtained.
From (-) 3-deoxyepiclausenamide: (-) epiclausenamide(-II₁) was obtained in 38% yield,m.p.107-109°C,[ α ] ²_{D}⁰ =-204(c 0.45 CH₃OH)
From (+) 3-deoxyepiclausenamide:(+) epiclausenamide(+II₁) was obtained in 28% yield, m.p.108-110°C,[ α ] ²_{D}⁰ =+201(c 0.25 CH₃OH)

### Example 19: Preparation of optical active cis neoclausemanide and cisepineoclausenamide II₇ Scheme 4)

(1) DEAD(diethyl azodicarboxylate) 2.10ml was added slowly to a solution of 1.5g (-) 3R4S5R neoclausenamidone, 2.6g of triphenylphosphine and 0.99g of ClCH₂COOH in 50ml of toluene. The reaction mixture was stirred at room temperature for 12hrs. And then treated with a mixture solvent of ethyl acetate and petroleum ether, and filtered. The filter cake was washed thoroughly with ethyl acetate. The combined filtrate and washing was evaporated. The residue with ethyl acetate/ petroleum ether was collected and the solvent was taken off by evaporation. The solid left behind without further purification was stirred with 750mg p-toluene sulfonic acid in 3ml of methanol at room temperature for 18hrs.The methanol was evaporated and the residue was dissolved in CH₂Cl₂.The organic layer was washed first with NaHCO₃ until the washing being slightly basic and then followed by saturated solution of NaCl. After taking off the solvent by evaporation, the residue was chromatographed on silica gel luted with ethyl acetate/petroleum ether (1:1) to give 1.05g (70%yield). After recrystallization from petroleum ether/ethyl acetate gave 0.7g of product (-)3S4S5R cisneoclausenamidone, m.p. 145.8-146.9°C,[ a ]²_{D}⁵ =-138.6(c 0.812, CHCl₃).
   By the same procedure described above (+) 3S4R5S cisneoclausenamidone yielded (=) 3R4R5S cisneoclausenamidone, m.p. 143.8-145.7°C,[ a ] ²_{D}⁵ =+139.8(c 1.74,CHCl₃).
(2). Reduction of C₆ ketone obtained from(1)
   To a solution of 0.15g (-) cisneoclusenamidone 3S4S5R in 20ml 0f methanol, o.o41g of NaBH4 was added gradually, with stirring after the reaction mixture was stirred at room temperature for three hours, the reaction mixture was neutralized with acetic acid. Then it was pound to a mixture of 50ml CH2CL2 and 20ml ice water. The organic layer was washed with saturated NaHCO3 aqueous solution and dried over Mg2SO4, After evaporating off the solvent. The white solid residue was chromatographed on silica geil to give 0.04g A after recrystallization from ether to give (-) cisneoclusenamide 3S4S5R6S mp. 168―170°C, [ α ] ³_{D}⁰ = -65.3 (C,0.32,MeoH) and from B, 0.075g of (-)cisepineoclusenamid 3S4S5R6R after recrystallization from methanol, with m.p. 271-273°C, [ α ] ³_{D}⁰ =-33.3 (C,0.34, DMSO).
   With the same procedure using (+) neoclusenamide give (+)cisneoclusenamide m.p. 164-166°C, ( α ) ³_{D}⁰ = +66.7(C, 0.32, MeoH, and (+) cisepineoclusenamide mp. 275-277°C, ( α ) ³_{D}⁰ = +31.2 (C,0.34, DMSO)

The following examples 20-24 are reference examples.

### Example 20: The mechanism of levoclausenamide in enhancing synaptic transmission in dentate gyrus of rats.

The rats were anaesthetized with urethane carbonate. The recording and stimulating electrodes were made from Teflon coated stainless steel wires. The bipolar stimulating electrode was sterotaxically placed in the entorhinal cortex to stimulate the perforant path, and the evoked potential was extracellular recorded from the dentate gyrus granule cell layer of ipsilateral. The population spike (PS) is served as an indication of granule cell's excitation. Long term potentiation (LTP) was induced by a 200 H_{z} high frequency stimulation 5 × single square wave pulses of 0.2 ms duration. The drug or vehicle (DMSO) were injected into lateral cerebral ventricle in a 5 µl volume over a 5 min period via a Hamilton syringe.
1. It was demonstrated in our study that both NMDA receptor and VDCC( Voltage-dependent calcium channel) involved in the LTP induced by high (200 H_{z}) frequency stimulation in the dentate gyrus of anesthetized rats, and the levoclausenamide-induced potentiation of synaptic transmission completely depended on VDCC. Accompanying these two kinds of potentiation of synaptic transmission, the activity of calcineurin and capain in the hippocampal and cortical tissues increased. These results (as shown in Tables 1 and 2) suggest that levoclausenamide potentiate the synaptic transmission in anesthetized rat through a mechanism different from that involved in HFS-induced LTP, but the phosphorylation and dephosphorylation of protein may be a common process involved in these two mechanisms.
2. It was demonstrated that few mossy fiber sprouting of CA3 and dentate gyrus of hippocampus in adult rat can be seen. However, levoclausenamide at dosage of 8 and 40 mg • kg⁻¹,orally-administrated daily for 10 days, dose-dependently increased the mossy fiber sprouting in the fascia dentate and stratum lucidum. But (+) clausenamide showed no significant effect on the mossy fiber sprouting in these two areas. This is morphological basis for levoclausenamide to incnease synaptic transmission (see Fig 1 and 2).
3.The cortical neurons and granule and pyramide cells in hippocampus of normal rats show a large of BDNF(brain derived neurotrophine factor). levoclausenamide (8, 40 mg • kg⁻¹) orally-administrated daily for 10 days, dose-dependently increased the BDNF protein expression in the cortical neurons and pyramide cells in the hippocampus, but (+) clausenamide showed slightly increasing effect only at a dose of 40 mg • kg⁻¹, suggesting that the different effects of (-), (+) clausenamide on hippocampal synaptic transmission may be related to their different actions on the BDNF protein expression. The result is shown in Fig 3.
3. Glutamate, the most abundant excitatory neurotransmitter in the CNS, released from presynaptic site and activated a variety of postsynaptic receptor, and then potentiated synaptic transmission. mGlu R (metabotropic glutamate receptor) indirectly regulates signaling and activats various second messenger cascades. The study on the effect of mGlu antagonist (±) MCPG ((±)-α -methyl-4-carboxyphenylglycine) on the (-) clausenamide-induced LTP in hippocampal DG in vivo showed that (±) MCPG(10 µmol, icv) inhibited the induction of LTP induced by HFS and levoclausenamide, and (±) MCPG 15min after HFS or levoclausenamide reversed the established LTP. These results syggested that MCPG-sensitive mGlu receptor was necessary for the induction and maintenance of LTP induced by levoclausenamide, and levoclausenamide enhanced synaptic transmission through mGlu receptor.

**Table 1 The calcineurin activity in the cortical and hippocampal tissue. The activity of calcineurin is expressed as (µmol Pi) /hr/ µ g protein.All the values are represented as mean±S.D. (n=5),* P <0.05 vs control group.**

| Groups# | Calcineurin | |
|---|---|---|
| | Cortical tissue | Hippocampal tissue |
| Control | 8.3±0.4 | 7.8±0.6 |
| APS | 7.9±0.7 | 8.1±0.7 |
| Nimodipine | 8.4±0.5 | 7.7±0.8 |
| HFS | 9.2±1.1 | 8.5±0.7 |
| AP5+HFS | 8.8±0.7 | 9.6±0.5* |
| Nimodipine+HFS | 7.7±1.4 | 9.8±0.4* |
| AP5+Nimodipine+HFS | 8.0±0.9 | 7.9±0.7 |
| (-)-Clausenamide | 11.2±0.9* | 10.4±0.5* |
| (-)-Clausenamide+AP5 | 11.6±1.4* | 10.1±0.8* |
| (-)-Clausenamide+Nimodipine | 9.1±1.2 | 8.3±0.6 |

| | | |
|---|---|---|
| # The dose of Nimodipine in related groups is 2nmol. | | |

**Table 2 The calpain activity in the cortical and hippocampal tissue. The activity of calpain is expressed as ΔA₅₉₅ₙₘ/hr/mg protein. All the values are represented as mean±S.D.(n=5).* P< 0.05 vs control group, # P<0.05 vs HFS group.**

| Groups# | Calcineurin | |
|---|---|---|
| | Cortical tissue | Hippocampal tissue |
| Control | 1.5±0.2 | 1.7±0.5 |
| AP5 | 1.6±0.4 | 1.6±0.6 |
| Nimodipine | 1.7±0.3 | 1.8±0.5 |
| HFS | 1.8±0.7 | 4.3±0.7** |
| AP5+HFS | 1.7±0.4 | 2.9±0.8*# |
| Nimodipine+HFS | 1.8±0.5 | 2.6±0.7*# |
| AP5+Nimodipine+HFS | 1.7±0.4 | 1.8±0.6 |
| (-)-Clausenamide | 2.6±0.5* | 2.7±0.8* |
| AP5+ (-)-Clausenamide Nimodipine | 2.7±0.6* | 2.6±0.7* |
| +(-)-Clausenamide | 1.6±0.6 | 1.9±0.6 |

| | | |
|---|---|---|
| # The dose of Nimodipine in related groups is 2nmol. | | |

### Example 21: Improving effect of (-) clausenamide on A β induced impairment of memory.

Pathological features of Alzheimer's disease (AD) include extensive neuronal loss and the presence of numerous neurofibrillary tangles and senile plaques in the brain. The senile plaques contain amyloid fibrils derived from a 39-43 amino acids peptide referred to as β-amyloid or Aβ which can produce neurotoxicity. Aβ 25-35 poccesses the similar neurotoxicity and ability of self-aggregation as Aβ 1-42. Administrated aggregated Aβ 25-35 into rat's lateral cerebral ventricle induced memory impairment in Morris water maze.
(-)clausenamide (8,40mg • kg⁻¹), administrated daily for 8 days starting from the 8^{th} day after icv injection of 15nmol aggregated Aβ 25-35, significantly ameliorated the spatial learning and memory impairment induced by aggregated A β 25-35. The latency for finding the platform beginning from 3^{th} to 5^{th} day in (-)clausenamide 8mg • kg⁻¹ and 40mg • kg⁻¹ groups was shorter than that of A β group. (+) clausenamide in doses of 8 and 40mg.kg⁻¹ and piracetam in dose of 400mg.kg⁻¹ showed no effect. With increasing of training days, the initial heading angle became small. On the 3^{th} day, the initial heading angle of A β group was larger than control. (-) clausenamide at doses of 8 and 40mg.kg⁻¹ significantly reduced the angle. (+) clausenamide at the same doses and piracetam at 400mg • kg⁻¹ showed no improving effects. (see Tab.3)

There are four strategies for finding platform in Morris water maze: random, circle, taxis and line. In the beginning of training the search strategies for finding target is mainly circle and random, with the increasing of training, the percentage of the two strategies declined gradually, Beginning from the 3^{th} day, declining speed in control and (-) clausenamide group was faster than A β ,(+) clausenamide and piracetam groups. Meanwhile, the appearance of taxis and line strategies in (-) clausenamide group increased markedly.

**Table 3a Effects of (-),(+) Clausenamide on the number of error of β-AP (25-35)-treated rats inone trial passive avoidance. Value is represented as MEAN±S.E.M.**

| Groups | Number of error | |
|---|---|---|
| | Training | Acquisition |
| Shan-operated+vehicle(n=8) | 1.6±0.42 | 0.3±0.12 |
| β-AP-created+vehicle(n=8) β | 2.6±0.71 | 0.9±0.33 |
| | 3.1±1.22 | 0.7±0.24 |
| -AP-treated+(-)Clausenamidc(8mg(kg,n=9) -AP-treated+(-)Clausenamide(40mg/kg,n=9 ) β | 4.4+0.87 | 1.1±0.27 |
| -AP-treated+(+)Clausenamide(8mg/kg,n=9) β | 3.3±0.42 | 0.8±0.38 |
| -AP-treated+(+)Clausenamide(40mg/kg,n=9 ) | 3.9±2.3 | 1.1±0.19 |
| β-AP-treated+piracetam(400mg/kg,n=8) | 4.1±1.14 | 1.6±0.26 |

**Tab 3b Effect of A β 25-35 on initial heading angle in Marris water mazw and the action of (-)clausenamide**

| Group | Training days | | | | |
|---|---|---|---|---|---|
| | 1 | 2 | 3 | 4 | 5 |
| A | 93.6±34.6 | 76.8±29.8 | 47.3±15.1 | 29.6±10.7 | 15.4±20.4 |
| B | 106.3±29.8 | 98.7±30.4 | 73.6±20.3* | 60.6±23.8* | 46.7±19.4* |
| C | 89.7+29.4 | 72.3+19.8 | 46.5±21.2# | 30.4±14.8# | 17.6±9.5# |
| D | 101.5±24.3 | 83.5±21.8 | 51.2±18.7# | 28.4±13.2# | 20.7±8.7# |
| E | 99.4±31.7 | 93.5±29.6 | 82.1±26.7 | 70.2±21.5 | 59.2±18.3 |
| F | 112.4±32.8 | 102.5±28.7 | 84.2±22.5 | 83.1±26.5 | 65.4±20.8 |
| G | 92.5±28.9 | 87.3±24.2 | 74.6±23.7 | 62.5±19.6 | 44.5±14.3 |

| | | | | | |
|---|---|---|---|---|---|
| *P<0.05 vs control; #P<0.05 vs A β 25-35 group. | | | | | |

A:control; B:A β 25-35, C:A β 25-35+(-)clausenanilde at 8mg/kg; D:A β 25-35+(-)clausenamide at 40mg/kg; E:A β 25-35+(+) clausenamide at 8mg/kg; F:A β 25-35+(+) clausenamide at 40mg/kg; G:A β 25-35+Piracetam at 400mg/kg

### Example 22: Inhibitory effect of (-) clausenamide on apoptosis in Bax α cDNA high expressing PC 12 cell line.

### 1.The construction of pcDNA3-bax α plasmid and its transfection into the PC12 cell line

In order to increase the expression level of the transfected gene, we first cut some of the 3' polyA signal sequences from the Baxα cDNA and constructed the intermediate plasmid pBluescript SK-Bax α (pA). Then cut the Bax α cDNA from the intermediate plasmid, religate with the eukaryotic expression plasmid pcDNA3 and obtained the pcDNA3-Bax α recombinant plasmid. The restriction enzyme analysis showed that the Bax α cDNA was inserted in right direction and has the same size. DNA sequences analysis showed that the inserted Bax α has the same nucleotide formation as the original cDNA. The recombinated Bax α has -50bp of 5' untranslated region(5'-UTR), 576 bp of open reading frame (ORF), and 137bp of 3' untranslated region (3'-UTR). The 5' methionine translation initiation region has the Kozak consensus sequences. So this plasmid may be used to transform the Bax α gene into the mammalian cells and guarantees highly expression of Bax α by using its strong promoter.

The Lipofectamine transfecting method was used to transfect the pcDNA3- Bax α into the PC12 cell line. G-418 was used to select the resistant cell clones. Western blot analysis and immunohistochemical method showed that the transfected PC-12 cells has a high expression of Bax α protein. This experiment successfully produced the Bax α high expressing PC12 cell line.

### 2.The inducement of Bax α high expressing PC12 cell apoptosis and the effect of (-) Clausenamide

Under normal condition, the Bax α high expressing cells had the same natural apoptosis rate with the neo transfected PC12 cells. It indicated that Bax α did not promote cell apoptosis without a death signal. After the 6-OHDA (100µM) treatment, TUNEL and the flowcytometry method showed that the Bax α high expressing cells had a higher apoptotic rate than those of the vector transfected controls (49.96% vs 32.9%), indicating that Bax α plays an important role in inducing apoptosis. (-) Clausenamide 10⁻⁷M, 10⁻⁶M, 10⁻⁵M could significantly lower the apoptotic rate from 49.9% to 36.23%, 28.1% and 9.5% seperately.

### 3. The mechanism of action of (-) Clausenamide in inhibiting apoptosis

24 hours after the 6-OHDA treatment, western blot analysis showed that the Bax α high expressing PC12 cells had a lower Bcl-2 level. (-) Clausenamide at to⁻⁷M, 10⁻⁶M could significantly increase the Bcl-2 expression level. Comparing with the vector transformed control, the mitochondria of the 6-OHDA treated Bax α high expressing cells had a lower level of GSH(9.84±1.20 vs 14.98±1.18 nmol/mg pro) and a higher level of MDA(2.46±0.19, 2.01±0.12 nmol/mg pro).(-) Clausenamide at 10⁻⁷M, 10⁻⁶M, 10⁻⁵M could significantly increase the mitochondrial GSH level (12.29 ± 0.99,15.10 ± 0.95,17.78 ± 1.04 nmol/mg pro )and lower the mitochondrial MDA level(1.76±0.17,1.54±0.13,1,33±0.11nmol/mg pro). It means that Bax α expression may promote the production of reactive oxygen species. The antiapoptotic effect of (-) clausenamide may be attributed to its reduction of the mitochondrial MDA level and the increase of mitochondrial GSH level. The increased mitochondrial GSH may be the result of the increased Bcl-2 level. As Bcl-2 itself is not an antioxidant, it may promote the closing of PT pores, and form heterodimers with Baxα and protect the mitochondria from injury and thus reduce the production of reactive oxygen, species. This kind of action is superior to those simple antioxidants.

The Bax α high expressing PC12 cells had a higher mitochondrial membrane potential in comparision with the neo transfected PC12 cells. After the 6-OHDA treatment for 12 hours, the Bax α high expressing PC12 cells showed a significantly low mitochondrial membrane potential (95.08 vs 24.6). (-) Clausenamide at 10⁻⁷M, 10⁻⁶M could increase the collapsed mitochondrial membrane potential significantly to 30.7±3.3 and 56.0±5.0. In comparison with the vector transformed control, the Bax α high expressing PC12 cells showed a significant decrease of the mitochondrial complex I and the complex IV activity (complex I: 1.44±0.16 vs 1.68±0.14umol/mg pro/min complex IV 0.09±0.019 vs 0.15±0.022 µmol pro/min). (-) Clausenamide at 10⁻⁷M, 10⁻⁶M, 10⁻⁵M could significantly increase the lowered mitochondrial complex I and complex IV activity (complex I:1.57±0.12, 1,98±0.05, 2.16 ±0.2 umol/mg pro/min; complex IV 0.12±0.015, 0.15±0.02, 0.18±0.01 umol/mg pro/min). Obviously, the increased mitochondrial membrane potential is considered to be one of the antiapoptotic mechanisms of (-) Clausenamide. The increased mitochondrial complex I and complex IV activity may account for the increased mitochondrial membrane potential. The Baxα protein may induce apoptosis by affecting enzyme activities of the mitochondrial electron transfer chain. And the mitochondrial complex I and complex IV could be the targeting points of the Bax α protein.

Studies have shown that the widely existing cytochrome c plays an important role in inducing apoptosis. Cytochrome c is the water soluble cytochrome within the cell and existed at the outside of the mitochondria inner membrane. Cytochrome c could be released into the cytoplasm from the mitochondrial to activate the apoptotic program. This study first purified the His-tagged Bax protein from the bacteria by the Metal Affinity method. When added to the isolated mitochondria, it caused a significant decrease of mitochondrial cytochrome c content (74.43±5.69ug/mg pro vs 50.2±3.65 ug/mg pro, p<0.01). (-)Clausenamide at concentrations of 10⁻⁷M, 10⁻⁶M, 10⁻⁵M could significantly decrease the Bax α induced cytochrome c release from mitochondria to 58.73 ±3.77 µg/mg pro, 61.7±5.3 µg/mg pro and 67.95±7.6 µg/mg pro separately. It means that (-) clausenamide may also act directly on mitochondrial itself to exert its antiapoptotic activity independent Bcl-2. The exact mechanism of action still remains to be determined.

In conclusion: (-) Clausenamide was able to inhibit the apoptosis of the PC12 Baxα cells caused by 6-OHDA. This effect may be related to its action in promoting Bcl-2 expression, raising the mitochondrial complex I and complex IV activity. (-) Clausenamide could also exerts its action on mitochondria itself by inhibiting the Bax α induced release of cytochrome c.

**Tab 4 Effects of (-) clausenamide on memory impairment induced by anisodine in step-down and step-through tests**

| Dugs | Doses mg/kg | Error number | | Latencies (sec) | |
|---|---|---|---|---|---|
| | | Step-through | Step-down | Step-through | Step-down |
| 0.9%Nacl | | 12.7±4.6 | 0.9±1.0 | 24.4±59 | 184±105 |
| Piracetam | 500 | 4.1±3.2*** | 0.7±0.5 | 107±110** | 180±110 |
| (-)Clausen-amide | 5 | 5.7±4.7*** | 0.5±0.5 | 47.9±90** | 186±129 |
| | 10 | 6.5±5.0*** | 0.2±0.4** | 59.4±91** | 296±97** |
| | 50 | 7.1±3.6*** | 0.2±0.4** | 17.1±12.4 | 265±91** |

| | | | | | |
|---|---|---|---|---|---|
| N=10,*X̅*±SD **P<0.05 | | | | | |
| ***P<0.01 vs 0.9%Nacl+anisodine | | | | | |

**Tab 5 Effects of piracetarn, (±), (-) clausenamide on memory in water maze test**

| Drugs | Doses (mg/kg) | Second day | 3^{th} day | 4^{th} day |
|---|---|---|---|---|
| | | Lalencies(sec) reaching platform | | |
| 0.9%Nacl | | 32.4±29.2 | 25.8±18.4 | 26.1±38.4 |
| Piracetam | 500 | 32.2±22.6 | 18.6±9.9** | 16.7±16.3** |
| (-)clausenamide | 10 | 28.7±35.2 | 17.4±10.9** | 12.1±6.9** |
| (±)clausenamide | 10 | 35.8±35.2 | 23.9±22.6 | 17.1±14.3 |
| | 50 | 36.6±37.0 | 25.7±13.8 | 14.6±10.4** |

| | | | | |
|---|---|---|---|---|
| N=10, *X*±SD ** P<0.05 vs 0.9%Nacl | | | | |

**Tab 6 Piracetam, (-), (±) clausenamide on memory impairment induced by anisodine in water maze test**

| Drugs | Animals number | Doses (mg/kg) | Errors number |
|---|---|---|---|
| Anisodine | 10 | | 15.9±11.3 |
| Piracetam | 9 | 500 | 23.5 ±25.0 |
| (-)clausenamide | 10 | 10 | 9.4±5.9** |
| (±)clausenamide | 8 | 10 | 16.6±23.9 |
| | 8 | 100 | 9.11±3.8** |

| | | | |
|---|---|---|---|
| ** P<0.05 vs anisodine group | | | |

**Tab 7 Effects of (-), (±) clausenamide on memory impairment induced by anisodine in step-through test**

| Drugs | Doses (mg/kg) | Latencies (Sec) | Error number |
|---|---|---|---|
| 0.9% Nacl | | 152.1±64.2 | 2.0±3.8 |
| Anisodine | 10 | 35.8±76.4** | 14.1±8.9## |
| (-)clausenamide | 10 | 138.2±86.7** | 6.7±11.0* |
| | 50 | 140.6±96.4** | 5.4±4.1** |
| (±)clausenamide | 10 | 43.0±65.8 | 11.3±21.5 |
| | 50 | 79.9±63.6 | 14.4±12.3 |

| | | | |
|---|---|---|---|
| N=10 * P<0.05 | | | |
| **P<0.01 vs anisodine | | | |
| ##P<0.01 vs 0.9% Nacl | | | |

### Example 23: comparison of improving memorial effects of (±), (-)Clausenamide

This invention used one trial avoidance task - step-down and step-through tests and water maze to compare the improving memorial effects of (±), (-) clausenamide.

The drugs were given orally once to animals before experiment in step-down and step-through tests. The water maze test lasted for 5 days in succession. The results were expressed as latency and errors number.

The results were shown in Tab.4 to Tab.7 All these results showed that the effective dosage for (-) clausenanide were 5,10,50mg.kg⁻¹.The effective dose of piracetam was 500mg.kg⁻¹. So the nootropic action of (-) clausenamide was 5-10 times and 50-100 times more potent than those of (±) clusenamide and piracetam.

### Example 24: Comparative study on the effects of (±) and (-) clausenamide on LTP.

Drugs or vehicle injection were delivered via cannula in the lateral cerebral ventricle at final concentration of 2×10⁻⁶mol/L. The population spike (PS) amplitude was employed as an indication of the level of excitation of the granular cell population in the dentate gyrus. To obtain this measurement, an evoked response was generated in the dentate gyrus granule cell layer by stimulating the pp at low freguency (0.033Hz) with, single square wave pulses of 0.15 ms duration The LTP formation must accord the following creteria: The PS amplitude increased is over 30% and this high PS amplitude can persist for 40 min.

Results showed that 30-60min after drug administration, (-)clusenamide increased PS amplitude by 48.5±81.2% and 38.9-63.4% in comparison with before drug administration and vehicle group, indicating that (-) clausenamide could increase basal synaptic transmission and induce LTP formation (see Tab 8).

(±) clausenamide at concentration of 2µM to 10µM showed inhibitory effect on PS amplitude. At time of 60min following(±) clausenamide, the PS amplitude decreased by 44 and 55% compared with that of (-) clausenamide. This indicated that (±) clausenamide at dosage of increasing 5 times could not induce LTP , but inhibit basic synaptic transmission(Fig. 6)

**Tab 8 Effects of (-) clausenamide ((-) CLA) and (+) clausenamide ((+) CLA) on basal synaptic transmission in dentate gyrus of hippocampus in anesthetized rats**

| Groups | Before drug | After drug | | | | | | | |
|---|---|---|---|---|---|---|---|---|---|
| | | 15' | PS% | 30' | PS% | 45' | PS% | 60' | PS% |
| Control | 103±3 | 110±9 | 6.7 | 108±7 | 4.9 | 110±10 | 6.7 | 112±7 | 8.7 |
| (-)CLA | 101±1 | 121±9 | 19.8 | 150±11 | 48.5 | 168±25 | 66.3 | 183±30 | 81.2 |
| PS% (+) | -1.9 | 10.0 | | 38.9 | | 52.7 | | 63.4 | |
| CLA | 101±3 | 107±8 | | 95±9 | -5.9 | 88±15 | -12.9 | 86±18 | -14.9 |
| PS% | -1.9 | -2.7 | | -12.0 | | -20.0 | | -23.2 | |

### Example 25

### Effect of four pairs optically active of "Clausenamide"on induction of LTP in hippocampal of rat

### Method:

Adult male SD rats were anesthetized to maintain a surgical level. A stainless steel cannula-recording electrode was placed in DG (dentate gyrus) of hippocampus using a stereotaxic instrument. The combination of cannula-recording electrode was constructed by insulating the outside of the cannula, except at the tip and top. In the top noninsulated area, a stainless steel wire was wrapped around the cannula and connected to the amplifier via an amphenol connector. The four pairs optically active clausenamides shown in table 9 was delivered by I.C.V. Responses were evoked via direct stimulation of the performant path (PP) using a stainless steel bipolar stimulating electrode. Constant current stimulation provided by a stimulator was delivered to the stimulating electrode through a Stimulus Isolation Unit.

Stimulation was delivered until antidromic spikes resulting from PP and stimulation were observed in the dentate. The stimulating electrode was adjusted until a characteristic EPSP was observed under constant current. The evoked responses were amplified on a series alternating current preamplifier, using a microcomputer, and then stored for off-line analysis using DataWave software. The current intensity that elicited a 50% maximal response in each animal was determined and used for all subsequent stimulation. To measure treatment effects, responses were evoked once every 30 sec throughout the entire experiment, and population spike amplitude(PSA) were calculated.

**table 9**

| Effect of four pairs optically active of "Clausenamide" on induction of LTP in hippocampal of rat | | | |
|---|---|---|---|
| **Treatment** | **PSA** | **(%), means±SD** | |
| | **15min** | **30min** | **60min** |
| reference (-) clausenamide | 131.8±0.4 | 138.5±8.9 | 158.1 ±4.2 |
| reference (+) clausenamide | 90.6±0.3 | 110.1±13.1 | 106.4±4.1 |
| (-) epiclausenamide | 97.8±21.7 | 91.2±22.5 | 117.3±22.5 |
| (+) epiclausenamide | 136.0±22.8 | 147.5±15.0 | 207.8±12.8 |
| (-) cisclausenamide | 126.2±2.3 | 141.2±8.6 | 205.4±24.7 |
| (+) cisclausenamide | 87.3±4.4 | 102.9±5.9 | 122.5±14.5 |
| (-) cisneoclausenamide | 94.1±6.7 | 98.2±6.6 | 94.9 |
| (+) cisneoclausenamide | 135.0±12.9 | 150.9±19.9 | 192.3±22.4 |

### Example 26

### Effects of Three Pairs Optically Active of "Clausenamide" on Colony -form Frequency in Human Embryonic Neural Stem Cells

### 1. Method

Human embryonic brain neural stem cells(NSC) were dissociated enzymatically with 0.05% trypsin-0.53 mM EDTA and cultured in the DMEM/F12 medium with N2 supplement (GIBCO), fibroblast growth factor-2 (20 ng/ml), epidermal growth factor (20 ng/ml) at 37°C. The media were changed once every week. The single cell suspension from neurosphere cells of NSC was dissociated by 0.1% trypsin. 2 X 10 ⁴ cells were seeded in a well of a 96-well plate. After 24 hours, the dose at 1 µM of tested chemical was added in a well. 6 chemicals, (-)neoclausenamide(-), (+)neoclausenamide(+), (-)epineoclausenamide(-), (+)epineoclausenamide(+), (-)cisneoclausenamide(-) and (+)cisneoclausenamide(+), were detected. 4 wells were used each group. The media with tested chemical were changed once every week. The colonies of NSC were formed and counted under a microscopy at 3 weeks, 4 weeks and 6 weeks.

### 2. Results

The results from Tab. X showed that the frequency of colonies of NSC was not significantly difference between control group and each group of 6 chemicals at 3 weeks and 4 weeks. At 6 weeks, there were significantly difference between control group and the group of (+)neoclausenamide(+), (-)epineoclausenamide(-), and (-)cisneoclausenamide(-), but there were not any difference between the control group and each group of the three chemicals such as (-)neoclausenamide(-), (+)epineoclausenamide(+), and (+)cisneo-clausenamide(+). Furthermore, the frequencies of NSC colonies of (+)neoclausenamide(+), (-)epineoclausenamide(-), and (-)cisneoclausenamide(-) were greatly higher than those of (-)neoclau-senamide(+), (+)epineoclausenamide(+), and (+)cisneoclau- senamide(+).

In conclusion, the three new chemicals, (+)neoclausenamide(+) II₂, (-)epineoclausenamide(-) II₃ and (-)cisneoclausenamide(-) II₆, could induce the increase of human embryonic neural stem cells. They may have the effects of promoting proliferation of NSC.

**Table 10.**

| Effects of Three Pairs Optically active "Clausenamides" on Colony - form Frequency in Human Embryonic Neural Stem Cells | | | | | | |
|---|---|---|---|---|---|---|
| No. | Group | N | Dose | 3 weeks | 4 weeks | 6 weeks |
| | | | µM | Colonies Mean± SD | Colonies Mean± SD | Colonies Mean± SD |
| | Control(DMSO) | 4 | 0 | 43.00 ±4.97 | 30.33 ±4.99 | 22.25 ±4.86 |
| 1.1 | (-)neoclausenamide(-) II₂ | 4 | 1 | 53.00 ±6.71 | 37.50 ±4.15 | 24.5 ±4.43 |
| 1.2 | (+)neoctausenamide(+) II₂ | 4 | 1 | 49.00 ±8.86 | 39.75 ±7.50 | 30.00 ±4.40 * |
| 2.1 | (-)epineoclausenamide(-) II₃ | 4 | 1 | 58.00 ±13.02 | 36.75 ±7.59 | 32.50± ±7.05 * |
| 2.2 | (+)epineoclausenamide(+) II₃ | 4 | 1 | 49.75 ±10.26 | 38.50 ±5.02 | 26.00 ±2.94 |
| 3.1 | (-)cisneoclausenamide(-) II₆ | 4 | 1 | 49.50 ±5.43 | 35.25 ±7.46 | 33.50 ±4.20 # |
| 3.2 | (+)cisneoclausenamide(+) II₆ | 4 | 1 | 47.00 ±9.03 | 42.50 ±10.01 | 24.00 ±4.69 |

| | | | | | | |
|---|---|---|---|---|---|---|
| * p<0.05(single tail T test); | | | | | | |
| # p<0.05(double tail T test) | | | | | | |

The present invention relates to some new optically active clausenamide derivatives, process of the preparation thereof, a pharmaceutical composition containing the same and their medical use, particularly their use in the respects of hypoxia protective, nootropic and neurodegenerative disease such as cerebral ischemia, Alzheimer disease and vascular dementia.

## Claims

1. Use of an optically active chiral compound of Formula II, 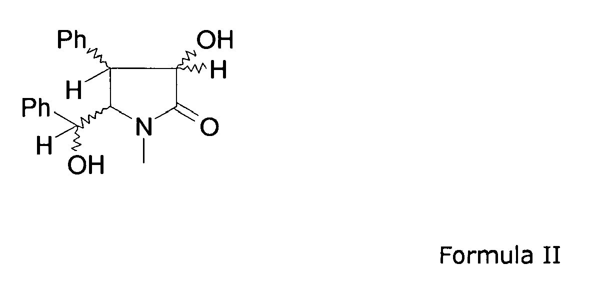 for the manufacture of a pharmacutical composition for the treatment of neurodegenerative diseases, wherein said derivative is selected from the group consisting of
3S4R5R6R (-) epiclauseanamide,
3R4S5S6S (+) epiclauseanamide,
3S4R5S6S (-) epineoclauseanamide
3R4S5R6R (+) epineoclauseanamide
3S4S5S6R (+) cisclauseanamide,
3R4R5R6S (-) cisclauseanamide,
3S4S5S6S (+) cisepiclauseanamide,
3R4R5R6R (-) cisepiclauseanamide,
3S4S5R6S (-) cisneoclauseanamide,
3R4R5S6R (+) cisneoclauseanamide,
3S4S5R6R (-) cisepineoclauseanamide and
3R4R5S6S (+) cisepineoclauseanamide.

2. Use according to claim 1, wherein the neurodegenerative disease is selected from the group consisting of cerebral ischemia, Alzheimer's disease or vascular dementia.

3. Pharmaceutical composition comprising an optically active compound as claimed in claim 1 together with at least one pharmaceutically acceptable carrier or excipient.

## Patentansprüche

1. Verwendung einer optisch aktiven chiralen Verbindung der Formel II, 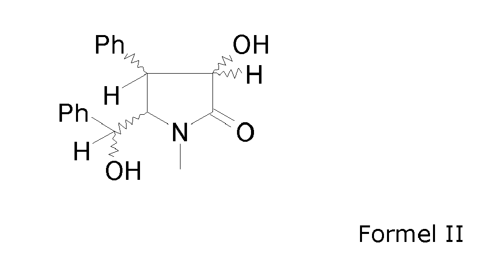 für die Herstellung einer pharmazeutischen Zusammensetzung für die Behandlung von neurodegenerativen Krankheiten, wobei das Derivat aus der Gruppe ausgewählt ist, die aus
3S4R5R6R (-) Epiclauseanamid,
3R4S5S6S (+) Epiclauseanamid,
3S4R5S6S (-) Epineoclauseanamid,
3R4S5R6R (+) Epineoclauseanamid,
3S4S5S6R (+) Cisclauseanamid,
3R4R5R6S (-) Cisclauseanamid,
3S4S5S6S (+) Cisepiclauseanamid,
3R4R5R6R (-) Cisepiclauseanamid,
3S4S5R6S (-) Cisneoclauseanamid,
3R4R5S6R (+) Cisneoclauseanamid,
3S4S5R6R (-) Cisepineoclauseanamid und
3R4R5S6S (+) Cisepineoclauseanamid besteht.

2. Verwendung nach Anspruch 1, wobei die neurodegenerative Krankheit aus der Gruppe ausgewählt ist, die aus zerebraler Ischemie, Alzheimersche Krankheit oder vaskulärer Demenz besteht.

3. Pharmazeutische Zusammensetzung, umfassend eine optisch aktive Verbindung nach Anspruch 1 zusammen mit zumindest einem pharmazeutisch akzeptablen Träger oder Hilfsstoff.

## Revendications

1. Utilisation d'un composé chiral optiquement actif de formule II, 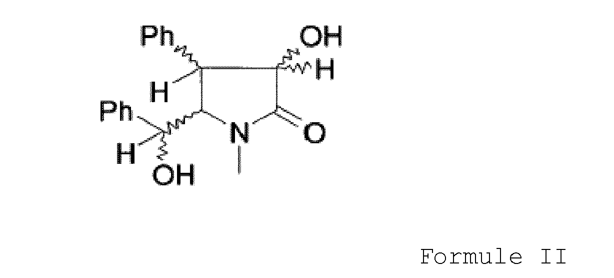 pour la fabrication d'une composition pharmaceutique destinée au traitement de maladies neurodégénératives, dans laquelle ledit dérivé est choisi dans le groupe constitué par
le 3S4R5R6R (-) épiclausenamide,
le 3R4S5S6S (+) épiclausenamide,
le 3S4R5S6S (-) épineoclausenamide
le 3R4S5R6R (+) épineoclausenamide
le 3S4S5S6R (+) cisclausenamide,
le 3R4R5R6S (-) cisclausenamide,
le 3S4S5S6S (+) cisépiclausenamide,
le 3R4R5R6R (-) cisépiclausenamide,
le 3S4S5R6S (-) cisneoclausenamide,
le 3R4R5S6R (+) cisneoclausenamide,
le 3S4S5R6R (-) cisépineoclausenamide et
le 3R4R5S6S (+) cisépineoclausenamide.

2. Utilisation selon la revendication 1, dans laquelle la maladie neurodégénérative est choisie dans le groupe constitué par une ischémie cérébrale, la maladie d'Alzheimer ou une démence vasculaire.

3. Composition pharmaceutique comprenant un composé optiquement actif tel que revendiqué selon la revendication 1, avec au moins un support ou un excipient pharmaceutiquement acceptable.
